# FASCICULE DE BREVET EUROPEEN

(11) **EP 0 790 053 B1**
(45) Date de publication et mention de la délivrance du brevet: **07.10.1998**
(21) Numéro de dépôt: 97400228.9
(22) Date de dépôt: 31.01.1997
(51) Int. Cl.: A61K 7/06

(54) **Utilisation d'un 2-amino-alcane-1,3-diol pour freiner la chute des cheveux et/ou induire et stimuler leur croissance**
Verwendung eines 2-Aminoalkan-1,3-Diol zur Verlangsamung des Haarausfalls und/oder zum Auslösen und Stimulieren des Haarwachstums
Use of 2-amino-alkane-1,3-diol to reduce hairloss and/or to induce and stimulate hairgrowth

(30) Priorité: 15.02.1996 FR 9601886
(43) Date de publication de la demande: 20.08.1997
(73) Titulaire: L'OREAL, 75008 Paris (FR)
(72) Inventeur: Fagot, Dominique, 75020 Paris (FR); Gaillard, Olivier, 75020 Paris (FR); Philippe, Michel, 91320 91320 Wissous (FR); Bernard, Bruno, 92200 Neuilly-Sur-Seine (FR)
(74) Mandataire: Tezier Herman, Béatrice

(56) Documents cités:
- EP-A- 0 278 505
- WO-A-92/16236
- WO-A-94/23694
- WO-A-95/03028
- FR-A- 2 718 960
- PATENT ABSTRACTS OF JAPAN vol. 13, no. 63 (C-568) [3411] & JP 63 255213 A (KANEBO)
- PATENT ABSTRACTS OF JAPAN vol. 13, no. 49 (C-565) [3397] & JP 63 243017 A (KANEBO)

## Description

La présente invention concerne l'utilisation, à titre de principe actif dans une composition cosmétique ou pour la préparation d'une composition pharmaceutique, d'une quantité efficace d'au moins un 2-amino-alcane-1,3-diol, ou de l'un de ses dérivés, destinée à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

On recherche depuis de nombreuses années, dans l'industrie cosmétique ou pharmaceutique, des substances permettant de supprimer ou de réduire l'effet de l'alopécie, et notamment de diminuer la chute des cheveux ou d'induire ou de stimuler leur croissance.

Dans cette optique, on a certes déjà proposé un grand nombre de substances ou de composés actifs très divers. On citera entre autres pour mémoire les vitamines comme la vitamine E, les acides aminés comme par exemple la sérine ou la méthionine, les vasodilatateurs comme l'acétylcholine et ses dérivés, les hormones femelles comme par exemple l'oestradiol, les agents kératolytiques comme l'acide salicylique ou des composés chimiques comme par exemple le 2,4-diamino 6-piperidinopyrimidine 3-oxyde ou "Minoxidil" décrit dans US 4 596 812 ou encore ses nombreux dérivés comme ceux décrits par exemple dans les demandes de brevet EP 353123, EP 356271, EP 408442, EP 522964, EP 420707, EP 459890, EP 519819.
On peut encore citer le 6-amino 1,2-dihydro 1-hydroxy 2-imino 4-pipéridino pyrimidine et ses dérivés, qui sont décrits plus particulièrement dans le brevet US-A- 4 139 619.

Il reste, d'une manière générale, qu'il serait intéressant et utile de pouvoir disposer de substances actives autres que celles déjà connues.
Le Minoxidil, s'il reste le composé de référence dans le domaine, présente des effets secondaires non négligeables qui rendent son utilisation délicate.
La découverte de substances ayant un effet sur la pousse et/ou la chute des cheveux sans présenter d'effets secondaires gênants reste un objectif majeur de la recherche.

Un des buts de la présente invention est donc de proposer une nouvelle façon de favoriser la survie et/ou la pousse des cheveux sans présenter d'effets secondaires néfastes.

De manière surprenante, la demanderesse a maintenant découvert que des composés de type 2-amino-alcane-1,3-diol ont un effet sur la survie et la croissance des cheveux.

Les composés de type 2-amino-alcane-1,3-diol ou leur dérivés font partie de ces composés des tissus que l'on nomme du terme très général de sphingolipides.
Parmi ces sphingolipides, les dérivés N-acylés à base sphinganine [(2S, 3R)-2-amino-1,3-octadécanediol] sont des céramides majoritairement présents dans le cheveu, alors que les dérivés analogues à base sphingénine [(2S, 3R, 4E)-2-amino-4-octadécène-1,3-diol), autres céramides, font partie des lipides majoritairement présents dans le stratum corneum de la peau.

Les céramides sont utilisés en cosmétique sous forme naturelle ou synthétique dans des compositions destinées par exemple à renforcer l'effet barrière du stratum corneum afin de réduire la perte en eau et donc le dessèchement de la peau (GB 2 178 312, GB 2 213 723, EP 227 994, EP 282 616, EP 556 957).
Ils sont également utilisés dans des compositions cosmétiques pour leurs propriétés conférant à la peau une meilleure élasticité (EP 500 437) ou encore dans des compositions à usage capillaire pour renforcer le cheveu et/ou réparer les dégâts causés par les agressions continuelles que celui-ci subit.

A la connaissance de la demanderesse, il n'a jamais été décrit ni même suggéré pour les 2-amino-alcane-1,3-diol, ou leurs dérivés, un effet sur la prolifération des cellules et encore moins des kératinocytes.
C'est sur la base de cette propriété nouvelle de ces amino-diols que la demanderesse a pu montrer que ces composés ont également un effet sur la survie du follicule pileux.
Ainsi, en augmentant le temps de survie du follicule pileux, on allonge la phase anagène du cycle pilaire ce qui a pour effet de retarder la chute des cheveux.

Parmi les sphingolipides, les composés à base sphingénine induisent l'apoptose, directement ou par une série d'événements impliquant des protéines cellulaires. Ce phénomène qui aboutit à la mort cellulaire, transposé au cycle pilaire, entraîne un arrêt de la croissance du follicule et une chute des cheveux.
La synthèse des composés à base sphingénine découle de plusieurs mécanismes distincts. On citera par exemple l'activation de la sphingomyélinase cellulaire par le facteur de nécrose tumorale de type α (TNFα), par la vitamine D, par l'interleukine 1, par l'antigène FAS ou les radiations ionisantes. On sait également que les composés à base sphingénine peuvent aussi être générés par l'activation de l'acyl-CoA sphinganine (sphingosine) N-acyltransférase (EC 2.3.1.24).

La demanderesse a montré que les composés de formule (I) peuvent interférer aussi bien au cours de la synthèse des composés à base sphingénine qu'au cours des événements induits par ces composés. Ceci a pour conséquence d'empêcher l'apoptose induite par les composés à base sphingénine et également de stimuler la croissance et la viabilité cellulaire.
Dans les traitements anticancéreux par chimiothérapie, l'utilisation d'agents anticancéreux entraîne au niveau du follicule pileux une mort cellulaire conduisant à la chute des cheveux. Cette alopécie induite est généralement transitoire mais partois peut être définitive (Hussein AM. South Med. J. 1993 ; 86 : 489-496). Cet effet secondaire conduit certains patients à refuser ce type de thérapie (Baxley K.O. et col. : Cancer Nurs. 1984 ; 7 : 499-503).
La doxorubicine, par exemple, induit une telle chute des cheveux. Or on sait que la doxorubicine active la céramide synthase, enzyme dont l'activation conduit à l'augmentation du taux intracellulaire de composés à base sphingénine qui eux-mêmes induisent le phénomène d'apoptose et de mort cellulaire.

La demanderesse a montré que l'utilisation de composés de formule (I) permet de contrecarrer l'effet secondaire néfaste des agents anticancéreux, comme par exemple la doxorubicine, en augmentant la viabilité cellulaire des kératinocytes des follicules pileux.

L'invention concerne l'utilisation dans une composition cosmétique ou pour la préparation d'une composition pharmaceutique en tant qu'agent favorisant la survie et/ou la pousse des cheveux, d'au moins un composé de formule générale (I) : 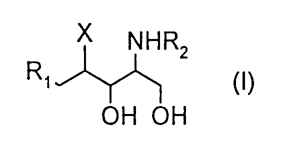
dans laquelle R₁ représente un radical hydrocarboné linéaire ou ramifié, éventuellement hydroxylé, saturé ou insaturé, ayant de 4 à 28 atomes de carbone,
R₂ représente un atome d'hydrogène ou le radical 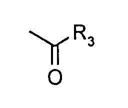
dans lequel R₃ représente un radical hydrocarboné linéaire ou ramifié, éventuellement hydroxylé, saturé ou insaturé, ayant de 1 à 29 atomes de carbone, l'hydroxyle pouvant être estérifié par une chaîne acyle ayant de 2 à 30 atomes de carbone, linéaire ou ramifiée, saturée ou insaturée ;
X représente un atome d'hydrogène ou le radical OH ;
ou d'au moins l'un de ses isomères optiques ou l'un des diastéréoisomères.
R₁ contient de préférence entre 11 et 23 atomes de carbone et encore plus préférentiellement 14 atomes de carbone.
R₂ représente de préférence le radical 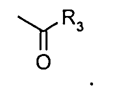
R₃ contient de préférence entre 7 et 25 atomes de carbone.

Préférentiellement, R₁ représente un radical hydrocarboné saturé et/ou linéaire plus particulièrement un radical hydrocarboné linéaire saturé.

Préférentiellement, R₁ représente un radical hydrocarboné saturé ayant 14 atomes de carbone, et encore plus préférentiellement R₁ représente un radical hydrocarboné linéaire, saturé, ayant 14 atomes de carbone.

L'agent favorisant la survie et/ou la pousse des cheveux peut être un mélange de composés de formule (I), pour lesquels R₁ et/ou R₂ sont des chaînes de longueurs différentes.

Ces composés peuvent être utilisés sous forme d'isomère pur ou de mélange d'isomères.

Ces composés présentent l'avantage de ne pas induire d'effets secondaires gênants, ce qui rend leur utilisation sans risque pour l'utilisateur.

On peut citer comme composés de formule (I) utilisables selon l'invention :
- le 2-amino-octadécane-1,3-diol,
- le 2-acétylamino-octadécane-1,3-diol,
- le 2-octanoylamino-octadécane-1,3-diol,
- le 2-tetracosanoylamino-octadécane-1,3-diol,
- le 2-N-(2-hydroxy-hexadécanoyl)-amino-octadécane-1,3-diol,
- le 2-oléoylamino-octadécane-1,3-diol,
- le 2-hexadécanoylamino-octadécane-1,3-diol,
- le 2-N-(2-hydroxy-docosanoyl)-amino-octadécane-1,3-diol,
- le 2-amino-octadécane-1,3,4-triol,
- le 2-hexadécanoylamino-octadécane-1,3,4-triol,
- le 2-N-(2-hydroxy-hexadécanoyl)-amino-octadécane-1,3,4-triol.

Préférentiellement, selon l'invention on utilise le 2-N-(2-hydroxy-hexadécanoyl)-amino-octadécane-1,3 diol et le 2-oléoylamino-octadécane-1,3-diol.

La quantité de composés de formule (I) utilisable selon l'invention dépend, bien entendu, de la nature du composé utilisé, de ses propriétés physico-chimiques et de son mode d'application. L'homme du métier sait ajuster la quantité de composé de formule (I) à utiliser selon les besoins.

A titre indicatif les composés de formule (I) peuvent être utilisés à des concentrations en poids comprises entre 10⁻⁴% et 20%, et de préférence entre 10⁻³ % et 10% dans la composition.

L'invention a également pour objet l'utilisation d'au moins un composé de formule (I) tel que défini précédemment pour combattre une alopécie induite par un traitement anticancéreux consistant en une chimiothérapie.

Lorsque le composé de formule (I) est utilisé dans une composition qui doit être appliquée sur la peau et particulièrement au niveau du cuir chevelu, cette composition peut se présenter sous toutes les formes galéniques normalement utilisées.

Pour une application topique sur la peau, la composition peut avoir la forme notamment d'une solution aqueuse, alcoolique, hydroalcoolique ou huileuse ou de dispersion du type lotion ou sérum, d'une émulsion de consistance liquide ou semi-liquide du type lait, obtenue par dispersion d'une phase grasse dans une phase aqueuse (H/E) ou inversement (E/H), ou d'une suspension ou d'une émulsion de consistance molle du type crème, mousse ou gel aqueux ou anhydre, ou encore de microcapsules ou microparticules, ou d'une dispersion vésiculaire de type ionique et/ou non ionique. Elle peut également prendre la forme d'une composition pour aérosol comprenant également un agent propulseur sous pression.

Quelque soit sa forme, cette composition est préparée selon les méthodes usuelles.

Les quantités des différents constituants de la composition selon l'invention sont celles classiquement utilisées dans les domaines considérés.

Le composé utilisé selon l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des shampooings antiparasitaires, etc.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

On peut également envisager que la composition comprenant au moins un composé de formule (I) à titre de principe actif destiné à induire et/ou stimuler la croissance des cheveux et/ou freiner leur chute soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

Cette composition peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides autres que les composés de formule (I), les huiles essentielles, l'acide salicylique et ses dérivés.

Il est possible aussi d'utiliser en association avec le composé de formule (I) utilisé selon l'invention des composés améliorant encore l'activité sur la repousse et/ou sur le ralentissement de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans US 4 596 812 ou encore ses nombreux dérivés, ou comme le 6-amino 1,2-dihydro 1-hydroxy 2-imino 4-pipéridinopyrimidine et ses dérivés tels que décrits dans le brevet US 4 139 619 ;
- les agents favorisant la repousse des cheveux comme ceux décrits par la demanderesse dans la demande de brevet européen publiée sous le numéro 0648488;
- les agents antibactériens tels que les macrolides, les pyranosides et les tétracyclines, et notamment l'érythromicine ;
- les agents antagonistes de calcium, comme la cinnarizine et le diltiazem ;
- des hormones, telles que l'estriol ou des analogues, ou la thyroxine et ses sels ;
- des agents anti-inflammatoires stéroïdiens, tels que les corticostéroïdes (par exemple : l'hydrocortisone) ;
- des agents antiandrogènes, tels que l'oxendolone, la spironolactone, le diéthylstilbestrol ;
- des antagonistes des 5-α-réductases ;
- des capteurs de radicaux OH, tels que le diméthylsulfoxyde ;

A la liste ci-dessus, d'autres composés peuvent également être rajoutés, à savoir par exemple le diazoxide, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés.

Selon l'invention on peut, entre autres, associer au moins un composé de formule (I) à d'autres agents actifs destinés notamment à la prévention et/ou au traitement des affections cutanées, particulièrement des affections du cuir chevelu. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromicine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acydovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ; - les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritant, en particulier vis à vis de composés irritants éventuellement présents dans les compositions.

Ainsi, un autre objet de l'invention concerne une composition comprenant une quantité efficace d'au moins un composé de formule (I) et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Dans les compositions selon l'invention, l'antagoniste de substance P, de CGRP ou de bradykinine ou l'inhibiteur de NO synthase préférentiellement utilisé est en une quantité allant de 0,000001 à 20 % du poids total de la composition, et en particulier en une quantité représentant de 0,0001 à 15 % du poids total de la composition.

Il est possible d'utiliser au moins un des composés répondant à la formule (I) dans la préparation d'une composition pharmaceutique, particulièrement dermatologique, destinée à traiter la chute et/ou à favoriser la repousse des cheveux.

En général, ces compositions pharmaceutiques se distinguent notamment des compositions cosmétiques par la quantité d'actif qu'elles contiennent. L'homme du métier, dans ce cas là sait déterminer la quantité d'actif utilisable en fonction du résultat recherché mais également du mode d'administration envisagé. A titre indicatif, le composé de formule (I) peut être utilisé dans la préparation d'une composition pharmaceutique à une concentration comprise entre 0,01% et 20% et préférentiellement entre 0,1% et 10% en poids par rapport au poids de la composition.

La composition cosmétique ou pharmaceutique selon l'invention peut être appliquée sur les zones alopéciques du cuir chevelu et des cheveux d'un individu, et éventuellement laissée en contact plusieurs heures et éventuellement rincée. On peut, par exemple, appliquer la composition contenant une quantité efficace d'au moins un composé de formule (I), le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition cosmétique comprenant une quantité efficace d'au moins un composé de formule (I), à laisser celle-ci en contact avec les cheveux et/ou le cuir chevelu, et éventuellement à rincer.

Les exemples et compositions suivants illustrent l'invention. Dans les compositions les proportions indiquées sont des pourcentages en poids sauf indications contraires.

### Exemple 1 : Mesure de l'effet prolifératif du 2-amino-octadécane-1,3-diol et de ses dérivés sur des kératinocytes en culture :

Des cellules HaCat (Boukamp et collaborateurs, J. Cell Biol. Vol. 106, Mars 1988, 761-771) sont cultivées dans du milieu DMEM (Société Gibco) contenant des acides aminés (mélange d'acides aminés non essentiels) à la concentration de 0,1 mM, de la pénicilline et de la streptomycine aux concentrations respectives de 50 Unités Internationales par millilitre et de 50 µg/ml, de la glutamine à la concentration de 2 mM, du pyruvate de sodium à la concentration de 1 mM et du sérum de veau foetal à 10%. Ces cellules ont été ensemencées à la densité de 10000 cellules par puits, dans les 24 puits de plaques du type multipuits (Costar, France).
24 heures après ensemencement, les cellules sont mises en contact avec les différents composés testés dans un milieu KBM (société Clonetics) contenant 0,15 mM d'ion calcium, de l'insuline à 5 µg/ml, de l'hydrocortisone à 0,5 µg/ml, et de la serumalbumine bovine délipidée à 1 µg/ml.
Un comptage cellulaire est réalisé 5 jours après l'addition des différents céramides grâce à un compteur de cellules du type Coulter Counter.
Les différents composés sont testés à 0,5 nM, 5 nM, 50 nM et 500 nM.
Les résultats exprimés en pourcentage représentent l'augmentation du nombre de cellules par rapport au contrôle, c'est à dire par rapport à une culture effectuée dans les mêmes conditions en l'absence de sphinganine.

Les différents composés testés sont :
A: 2-amino-octadécane-1,3-diol,
B: 2-oleoylamino-octadécane-1,3-diol,
C: 2-N-(2-hydroxy-hexadécanoyl)-amino-octadécane-1,3-diol,
D : 2-tétracosanoylamino-octadécane-1,3-diol,
E : 2-acétylamino-octadécane-1,3-diol,
F : 2-octanoylamino-octadécane-1,3-diol.

| | A | B | C | D | E | F |
|---|---|---|---|---|---|---|
| 0,5 nM | 7 | 17 | 26 | 22 | 17 | 15 |
| 5 nM | 15 | 24 | 36 | 27 | 34 | 18 |
| 50 nM | 17 | 23 | 42 | 33 | 29 | 31 |
| 500 nM | 30 | 14 | 22 | 42 | 35 | 27 |

Ces résultats montrent que le 2-amino-octadécane-1,3-diol et ses dérivés ont une activité proliférative sur les kératinocytes en culture.

### Exemple 2 : Mesure de l'effet du 2-amino-octadécane-1,3-diol et de ses dérivés sur la viabilité cellulaire :

Des cellules HaCat sont cultivées comme précédemment.
Elles sont ensuite ensemencées à la densité de 20000 cellules par puits, dans les 24 puits de plaques du type multipuits (Costar, France).
24 heures après ensemencement, les cellules sont mises en contact avec les différents composés testés dans un milieu KBM (société Clonetics) contenant de l'insuline à 5 µg/ml et de l'hydrocortisone à 0,5 µg/ml.
La viabilité cellulaire est évaluée 48 heures plus tard par la mesure de la respiration mitochondriale. Celle-ci est réalisée à l'aide d'un kit XTT, vendu par la société Boehringer et selon les prescriptions du fournisseur.

Les composés A, B et C de l'exemple précédent ont été testés à la concentration de 50 nM, de même qu'un dérivé à base sphingénine : la N-palmitoylsphingénine de chez Sigma (C16H).

Les résultats exprimés en pourcentage représentent l'augmentation du nombre de cellules par rapport au contrôle, c'est à dire par rapport à une culture effectuée dans les mêmes conditions en l'absence de composés alcane-diol .

| Contrôle | 0 |
|---|---|
| A | +17 |
| B | +23 |
| C | +20 |
| C16H | -25 |

Les résultats montrent que le 2-amino-octadécane-1,3-diol (A), le 2-oleoylamino-octadécane-1,3-diol (B) et le 2-N-(2-hydroxy-hexadécanoyl)amino-octadécane-1,3-diol (C) augmentent la viabilité des kératinocytes en culture, alors que la N-palmitoylsphingénine (C16H) a un effet cyto-toxique sur les cellules en culture.
Ce résultat montre que les composés à base sphingénine ne peuvent être utilisés pour le traitement des follicules pileux puisqu'ils entraînent en culture la mort des cellules.

### Exemple 3 : Mesure de l'effet du 2-amino-octadécane-1,3-diol et de ses dérivés sur le cycle cellulaire :

Des cellules HaCat sont cultivées comme précédemment.
Elles sont ensuite ensemencées à la densité de 20000 cellules par puits, dans les 24 puits de plaques du type multipuits (Costar, France).
24 heures après ensemencement, les cellules sont mises en contact avec les différents composés testés dans un milieu KBM (société Clonetics) contenant de l'insuline à 5 µg/ml et de l'hydrocortisone à 0,5 µg/ml et du 5-bromo-2' deoxyuridine (BrdU) à 1µ.M
La phase du cycle cellulaire est évaluée 48 heures plus tard par la mesure de l'incorporation de BrdU dans l'acide désoxyribonucléique génomique. Celle-ci est réalisée à l'aide d'un kit "BrdU fast", vendu par la société Boehringer et selon les prescriptions du fournisseur.

Les composés A, B et C de l'exemple précédent ont été testés à la concentration de 50 nM.

Les résultats exprimés en pourcentage représentent l'augmentation du nombre de cellules entrées en phase S par rapport au contrôle, c'est à dire par rapport à une culture effectuée dans les mêmes conditions en l'absence de composés alcane-diol.

| | 72 heures | 96 heures |
|---|---|---|
| Contrôle | 0 | 0 |
| A | 36 | 41 |
| B | 43 | 51 |
| C | 17 | 43 |

Les résultats montrent que la 2-amino-octadécane-1,3-diol (A), le 2-oleoylamino-octadécane-1,3-diol (B) et le 2-N-(2-hydroxy-hexadécanoyl)amino-octadécane-1,3-diol (C) augmentent le nombre de kératinocytes entrés en phase S, indiquant ainsi une stimulation de l'activité mitotique cellulaire.

### Exemple 4 : Mesure de l'effet du 2-amino-octadécane-1,3-diol et de ses dérivés sur la survie du follicule pileux in vitro :

Des follicules pileux viables *in vitro* sont préparés selon la technique décrite dans la demande de brevet n° 95-08465, déposée en France le 12 juillet 1995 par la demanderesse.

A partir d'une biopsie de scalp, une bande de cuir chevelu assez fine est isolée à l'aide d'un scalpel. Avec une micropince, le tissu adipeux entourant les follicules est éliminé, en évitant d'endommager le bulbe pilaire. Sous un microscope, le follicule est coupé avec un scalpel pour le séparer de son environnement épidermique et dermique.

Le fragment obtenu a été maintenu en culture dans du milieu Williams E, à 37°C, en atmosphère humide en présence de 5% de CO₂.

La survie des follicules est évaluée chaque jour par décompte des follicules vivants, selon leur aspect morphologique.

Les résultats sont donnés en pourcentage de follicules survivants par jour.

L'expérience a été réalisée avec les composés suivants à la concentration de 50 nM :
B : 2-amino-octadécane-1,3-diol,
C : 2-oleoylamino-octadécane-1,3-diol,
D : 2-N-(2-hydroxy-hexadécanoyl)-amino-octadécane-1,3-diol,
en comparaison avec le milieu de culture sans alcane-diol (A).

| Jours | 0 | 1 | 5 | 10 | 15 | 20 | 25 |
|---|---|---|---|---|---|---|---|
| A n=19 | 100 | 100 | 84 | 16 | 0 | 0 | 0 |
| B n=10 | 100 | 100 | 90 | 20 | 0 | 0 | 0 |
| C n=9 | 100 | 100 | 100 | 78 | 33 | 0 | 0 |
| D n=12 | 100 | 100 | 100 | 50 | 42 | 25 | 0 |
| n = nombre de follicules testés dans l'expérience. | | | | | | | |

Cette expérience montre que les alcanes-diols augmentent le temps de survie du follicule pileux en culture.

### Exemple 5 : Effet du 2-amino-octadécane-1,3-diol et de ses dérivés sur la viabilité cellulaire en présence de doxorubicine.

Des cellules HaCat sont cultivées comme dans l'exemple 2.
24 heures après ensemencement, les cellules sont mises en contact avec les différents composés testés dans un milieu KBM (société Clonetics) contenant de l'insuline à 5 µg/ml et de l'hydrocortisone à 0,5 µg/ml.
Les cellules sont cultivées en présence du 2-amino-octadécane-1,3-diol à tester jusqu'à l'évaluation de la viabilité cellulaire.
La doxorubicine à la concentration de 1µg/ml est ajoutée au milieu de culture pendant 2 heures au début de l'expérience.
La viabilité cellulaire est évaluée 48 heures plus tard par la mesure de la respiration mitochondriale. Celle-ci est réalisée à l'aide d'un kit XTT, vendu par la société Boehringer et selon les prescriptions du fournisseur.

L'expérience a été réalisée avec les composés suivants à la concentration de 50 nM :
C: 2-amino-octadécane-1,3-diol,
D: 2-oleoylamino-octadécane-1,3-diol,
E: 2-N-(2-hydroxy-hexadécanoyl)-amino-octadécane-1,3-diol,
en comparaison avec le milieu de culture sans alcane-diol et sans doxorubicine (A), et avec un milieu ne contenant que de la doxorubicine (B).

Les résultats exprimés en pourcentage, représentent l'augmentation du nombre de cellules par rapport au contrôle (A), c'est à dire par rapport à une culture effectuée dans les mêmes conditions en l'absence de composés alcane-diol et de doxorubicine.

| A | 0 |
|---|---|
| B | -20 |
| C | +25 |
| D | +28 |
| E | +23 |

La doxorubicine entraîne une diminution de la viabilité cellulaire. La présence dans le milieu de culture d'un 2-amino-octadécane-1,3-diol inhibe cet effet et augmente la viabilité cellulaire.

### Exemple 6 : Effet du 2-amino-octadécane-1,3-diol et de ses dérivés sur la viabilité cellulaire en présence d'un composé à base sphingénine la N-acétyl sphingénine.

Les cellules sont cultivées comme dans l'exemple 5.
La N-acétyl sphingénine est à la concentration de 10µM.

L'expérience a été réalisée avec les composés suivants à la concentration de 50 nM :
C : 2-amino-octadécane-1,3-diol,
D: 2-oleoylamino-octadécane-1,3-diol,
E : 2-N-(2-hydroxy-hexadécanoyl)-amino-octadécane-1,3-diol,
en comparaison avec le milieu de culture sans alcane-diol et sans N-acétyl sphingénine (A), et avec un milieu ne contenant que de la N-acétyl sphingénine (B).

Les résultats exprimés en pourcentage représentent le nombre de cellules par rapport au contrôle (A), c'est à dire par rapport à une culture effectuée dans les mêmes conditions en l'absence de composés alcane-diol et de N-acétyl sphingénine.

| A | 100 |
|---|---|
| B | 57 |
| C | 72 |
| D | 76 |
| E | 78 |

La N-acétyl sphingénine entraîne une diminution de la viabilité cellulaire. La présence dans le milieu de culture d'un 2-amino-octadécane-1,3-diol tend à contrer cet effet.

### Exemple 7 : Exemples de compositions à appliquer sur les cheveux et/ou le cuir chevelu. Ces compositions peuvent se préparer par simple mélange (M.A.: Matière Active).

| Soin après-shampooing à rincer : | |
|---|---|
| Rewoquat W75PG* | 2 g M.A. |
| 2-amino-octadécane-1,3-diol | 5 g |
| Mélange d'alcool cétylique et cétyl stéarylique oxyéthyléné | 3 g |

| Conservateur, parfum | |
|---|---|
| Eau q.s.p. | 100 g |

| | |
|---|---|
| * : Méthosulfate de 1-méthyl 2-suif 3-suifamidoéthylimidazolium/propylène glycol (75/25) vendu par Witco. | |

| Shampooing : | |
|---|---|
| Lauryl éther sulfate de sodium (28% M.A.) | 60 g |
| Cocoylbétaïne | 9 g |
| 2-oleoylamino-octadécane-1,3-diol | 4 g |

| Conservateur, parfum | |
|---|---|
| Eau q.s.p. | 100 g |
| HCl q.s. | pH 6 |

| Shampooing traitant : | |
|---|---|
| Lauryl éther sulfate de sodium (28%M.A.) | 75 g |
| Empilan CIS** | 1 g |
| 2-N-(2-hydroxy-hexadécanoyl)-amino-octadécane-1,3-diol | 1 g |
| Eau q.s.p. | 100 g |

| | |
|---|---|
| ** : Monoisopropanolamide d'acide de coprah vendu par Albright et Wilson. | |

| Lotion à rincer : | |
|---|---|
| Catinal DC50®*** | 0,5 g M.A. |
| 2-N-(2-hydroxy-hexadécanoyl)-amino-octadécane-1,3-diol | 4 g |

| Conservateur, parfum | |
|---|---|
| Eau q.s.p. | 100 g |
| NaOH q.s. | pH 5,5 |

| | |
|---|---|
| *** : Chlorure de béhényltriméthylammonium à 80% dans un mélange eau/isopropanol (15/85) vendu par Toho. | |

| Gel aqueux : | |
|---|---|
| Carbopol 940® vendu par Goodrich | 0,6 g |
| Transcutol® vendu par Gattefosse | 5,0 g |
| Triéthanolamine | 0,3 g |
| Conservateurs | 0,3 g |
| Propylène glycol | 3,0 g |
| NaOH | 0,007 g |
| 2-oleoylamino-octadécane-1,3-diol | 4 g |
| Eau q.s.p. | 100 g |

| Lotion : | |
|---|---|
| -2-acétylamino-octadécane-1,3-diol | 0,2 g |
| - Gantrez es 425**** | 1 g M.A. |
| - Celquat I200***** | 0,5 g |
| - Ethanol | 50 g |
| - Eau déminéralisée q.s.p. | 100 g |

| | |
|---|---|
| **** : Copolymère méthylvinyléther/anhydride maléique mono estérifié vendu par la société ISP. | |
| ***** : Copolymère d'hydroxyéthylcellulose et de chlorure de diallyldiméthyl ammonium vendu par la société NATIONAL STARCH. | |

## Revendications

1. Utilisation dans une composition cosmétique ou pour la préparation d'une composition pharmaceutique destinée à favoriser la survie et/ou la pousse des cheveux, d'une quantité efficace d'au moins un composé de formule générale (I) : 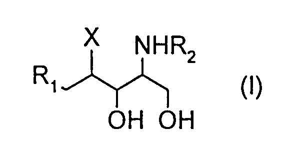
dans laquelle R₁ représente un radical hydrocarboné linéaire ou ramifié, éventuellement hydroxylé, saturé ou insaturé, ayant de 4 à 28 atomes de carbone ;
R₂ représente un atome d'hydrogène ou le radical 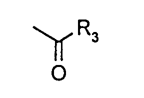
dans lequel R₃ représente un radical hydrocarboné linéaire ou ramifié, éventuellement hydroxylé, saturé ou insaturé, ayant de 1 à 29 atomes de carbone, l'hydroxyle pouvant être estérifié par une chaîne acyle ayant de 2 à 30 atomes de carbone, linéaire ou ramifiée, saturée ou insaturée ;
X représente un atome d'hydrogène ou le radical OH ;
ou d'au moins l'un de ses isomères optiques ou l'un des diastéréoisomères.

2. Utilisation selon la revendication 1, caractérisée par le fait que R₁ représente un radical hydrocarboné présentant entre 11 et 23 atomes de carbone.

3. Utilisation selon l'une quelconque des revendications 1 ou 2, caractérisée par le fait que R₁ représente un radical hydrocarboné saturé.

4. Utilisation selon l'une quelconque des revendications précédentes, caractérisée par le fait que R₁ présente 14 atomes de carbone.

5. Utilisation selon l'une quelconque des revendications 1 à 4, caractérisée par le fait que R₁ représente un radical hydrocarboné linéaire.

6. Utilisation selon l'une quelconque des revendications précédentes, caractérisée par le fait que R₂ est un radical

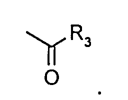

7. Utilisation selon l'une quelconque des revendications précédentes, caractérisée par le fait que R₃ est un radical hydrocarboné ayant de 7 à 25 atomes de carbone.

8. Utilisation selon l'une quelconque des revendications précédentes, caractérisée par le fait que la quantité de composé de formule (I) est comprise entre 10⁻⁴% et 20% en poids par rapport au poids de la composition.

9. Utilisation selon la revendication précédente, caractérisée par le fait que la quantité de composé de formule (I) est comprise entre 10⁻³% et 10% en poids par rapport au poids de la composition.

10. Utilisation selon l'une quelconque des revendications précédentes, caractérisée par le fait que le composé de formule (I) est choisi parmi :
- le 2-amino-octadécane-1,3-diol,
- le 2-acétylamino-octadécane-1,3-diol,
- le 2-octanoylamino-octadécane-1,3-diol,
- le 2-tetracosanoylamino-octadécane-1,3-diol,
- le 2-N-(2-hydroxy-hexadécanoyl)-amino-octadécane-1,3-diol,
- le 2-oléoylamino-octadécane-1,3-diol,
- le 2-hexadécanoylamino-octadécane-1,3-diol,
- le 2-N-(2-hydroxy-docosanoyl)-amino-octadécane-1,3-diol,
- le 2-amino-octadécane-1,3,4-triol,
- le 2-hexadécanoylamino-octadécane-1,3,4-triol,
- le 2-N-(2-hydroxy-hexadécanoyl)-amino-octadécane-1,3,4-triol.

11. Utilisation selon la revendication précédente, caractérisée par le fait que le composé de formule (I) est le 2-N-(2-hydroxy-hexadécanoyl)-amino-octadécane-1,3-diol ou le 2-oléoylamino-octadécane-1,3-diol.

12. Utilisation selon l'une quelconque des revendications précédentes pour combattre une alopécie induite par un traitement anticancéreux consistant en une chimiothérapie.

13. Composition cosmétique ou pharmaceutique comprenant, dans un milieu cosmétiquement ou pharmaceutiquement acceptable, au moins un composé tel que défini dans l'une quelconque des revendications 1 à 11 et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

14. Composition selon la revendication précédente, caractérisée en ce que l'antagoniste de substance P, de CGRP ou de bradykinine ou l'inhibiteur de NO synthase est utilisé en une quantité allant de 0,000001 à 20 % du poids total de la composition, et en particulier en une quantité représentant de 0,0001 à 15 % du poids total de la composition.

15. Procédé de traitement cosmétique des cheveux et/ou du cuir chevelu, caractérisé par le fait qu'il consiste à appliquer sur les cheveux et/ou le cuir chevelu, une composition cosmétique telle que définie dans l'une quelconque des revendications 13 et 14, à laisser celle-ci en contact avec les cheveux et/ou le cuir chevelu, et éventuellement à rincer.

## Claims

1. Use, in a cosmetic composition or for the preparation of a pharmaceutical composition intended to promote hair survival and/or growth, of an effective amount of at least one compound of general formula (I): 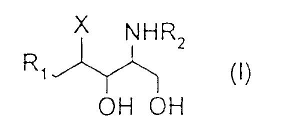
in which R₁ represents a saturated or unsaturated, optionally hydroxylated, linear or branched hydrocarbon radical having from 4 to 28 carbon atoms,
R₂ represents a hydrogen atom or the radical 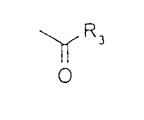
in which R₃ represents a saturated or unsaturated, optionally hydroxylated, linear or branched hydrocarbon radical having from 1 to 29 carbon atoms, it being possible for the hydroxyl to be esterified by a saturated or unsaturated, linear or branched acyl chain having from 2 to 30 carbon atoms,
X represents a hydrogen atom or the OH radical,
or of at least one of its optical isomers or one of the diastereoisomers.

2. Use according to claim 1, characterized in that R₁ represents a hydrocarbon radical having between 11 and 23 carbon atoms.

3. Use according to either of Claims 1 and 2, characterized in that R₁ represents a saturated hydrocarbon radical.

4. Use according to any one of the preceding claims, characterized in that R₁ has 14 carbon atoms.

5. Use according to any one of Claims 1 to 4, characterized in that R₁ represents a linear hydrocarbon radical.

6. Use according to any one of the preceding claims, characterized in that R₂ is a radical

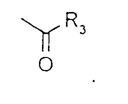

7. Use according to any one of the preceding claims, characterized in that R₃ is a hydrocarbon radical having from 7 to 25 carbon atoms.

8. Use according to any one of the preceding claims, characterized in that the amount of compound of formula (I) is between 10⁻⁴% and 20% by weight with respect to the weight of the composition.

9. Use according to the preceding claim, characterized in that the amount of compound of formula (I) is between 10⁻³% and 10% by weight with respect to the weight of the composition.

10. Use according to any one of the preceding claims, characterized in that the compound of formula (I) is chosen from:
- 2-amino-1,3-octadecanediol,
- 2-acetylamino-1,3-octadecanediol,
- 2-octanoylamino-1,3-octadecanediol,
- 2-tetracosanoylamino-1,3-octadecanediol,
- 2-N-(2-hydroxyhexadecanoyl)amino-1,3-octadecanediol,
- 2-oleoylamino-1,3-octadecanediol,
- 2-hexadecanoylamino-1,3-octadecanediol,
- 2-N-(2-hydroxydocosanoyl)amino-1,3-octadecanediol,
- 2-amino-1,3,4-octadecanetriol,
- 2-hexadecanoylamino-1,3,4-octadecanetriol,
- 2-N-(2-hydroxyhexadecanoyl)amino-1,3,4-octadecanetriol.

11. Use according to the preceding claim, characterized in that the compound of formula (I) is 2-N-(2-hydroxyhexadecanoyl)amino-1,3-octadecanediol or 2-oleoylamino-1,3-octadecanediol.

12. Use according to any one of the preceding claims for combating an alopecia induced by an anticancer treatment consisting of a chemotherapy.

13. Cosmetic or pharmaceutical composition comprising, in a cosmetically or pharmaceutically acceptable medium, at least one compound as defined in any one of Claims 1 to 11 and at least one agent chosen from antibacterial agents, agents for combating parasites, antifungal agents, antiviral agents, anti-inflammatory agents, antipruriginous agents, anaesthetic agents, keratolytic agents, agents for combating free radicals, antiseborrhoeic agents, antidandruff agents, antiacne agents, agents which modulate cutaneous pigmentation and/or proliferation and/or differentiation, substance P, CGRP or bradykinin antagonists or NO synthase inhibitors.

14. Composition according to the preceding claim, characterized in that the substance P, CGRP or bradykinin antagonist or the NO synthase inhibitor is used in an amount ranging from 0.000001 to 20% of the total weight of the composition and in particular in an amount representing from 0.0001 to 15% of the total weight of the composition.

15. Process for the cosmetic treatment of the hair and/or of the scalp, characterized in that it consists in applying on the hair and/or the scalp a cosmetic composition as defined in either one of Claims 13 and 14, in leaving this composition in contact with the hair and/or the scalp, and optionally in rinsing.

## Patentansprüche

1. Verwendung in einer kosmetischen Zusammensetzung oder für die Herstellung einer pharmazeutischen Zusammensetzung als Mittel, das die Lebensdauer der Haare verlängert und/oder das Haarwachstum fördert, einer wirksamen Menge mindestens einer Verbindung der allgemeinen Formel (I), 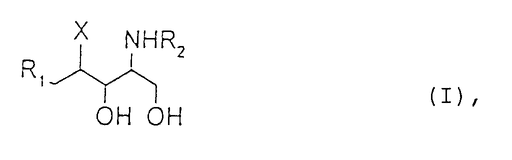 in der bedeuten:
- R₁ eine geradkettige oder verzweigte, gegebenenfalls hydroxylierte, gesättigte oder ungesättigte Kohlenwasserstoffgruppe mit 4 bis 28 Kohlenstoffatomen,
- R₂ ein Wasserstoffatom oder die Gruppe 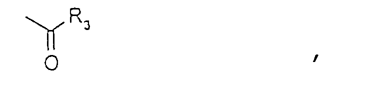 in der R₃ eine geradkettige oder verzweigte, gegebenenfalls hydroxylierte, gesättigte oder ungesättigte Kohlenwasserstoffgruppe mit 1 bis 29 Kohlenstoffatomen bedeutet, wobei der Hydroxyrest mit einer geradkettigen oder verzweigten, gesättigten oder ungesättigten Acylkette mit 2 bis 30 Kohlenstoffatomen verestert sein kann,
- X ein Wasserstoffatom oder eine HydroxyGruppe;
oder mindestens eines ihrer optisch aktiven Isomeren oder eines der Diasteromeren.

2. Verwendung nach Anspruch 1, dadurch gekennzeichnet, daß R₁ eine Kohlenwasserstoffgruppe ist, die 11 bis 23 Kohlenstoffatome enthält.

3. Verwendung nach einem der Ansprüche 1 und 2, dadurch gekennzeichnet, daß R₁ eine gesättigte Kohlenwasserstoffgruppe ist.

4. Verwendung nach einem der vorhergehenden Ansprüche, dadurch gekennzeichnet, daß R₁ 14 Kohlenstoffatome aufweist.

5. Verwendung nach einem der Ansprüche 1 bis 4, dadurch gekennzeichnet, daß R₁ eine geradkettige Kohlenwasserstoffgruppe ist.

6. Verwendung nach einem der vorhergehenden Ansprüche, dadurch gekennzeichnet, daß R₂ eine Gruppe 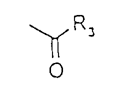 ist.

7. Verwendung nach einem der vorhergehenden Ansprüche, dadurch gekennzeichnet, daß R₃ eine Kohlenwasserstoffgruppe ist, die 7 bis 25 Kohlenstoffatome aufweist.

8. Verwendung nach einem der vorhergehenden Ansprüche, dadurch gekennzeichnet, daß der Mengenanteil der Verbindung der Formel (I) 10⁻⁴ % bis 20 Gew.-%, bezogen auf das Gewicht der Zusammensetzung, beträgt.

9. Verwendung nach dem vorhergehenden Anspruch, dadurch gekennzeichnet, daß der Mengenanteil der Verbindung der Formel (I) 10⁻³ bis 10 Gew.-%, bezogen auf das Gewicht der Zusammensetzung, beträgt.

10. Verwendung nach einem der vorhergehenden Ansprüche, dadurch gekennzeichnet, daß die Verbindung der Formel (I) unter
- 2-Aminooctadecan-1,3-diol,
- 2-Acetylaminooctadecan-1,3-diol,
- 2-Octanoylaminooctadecan-1,3-diol,
- 2-Tetracosanoylaminooctadecan-1,3-diol,
- 2-N-(2-Hydroxyhexadecanoyl)-aminooctadecan-1,3-diol,
- 2-Oleoylaminooctadecan-1,3-diol,
- 2-Hexadecanoylaminooctadecan-1,3-diol,
- 2-N-(2-Hydroxydocosanoyl)-aminooctadecan-1,3-diol,
- 2-Aminooctadecan-1,3,4-triol,
- 2-Hexadecanoylaminooctadecan-1,3,4-triol,
- 2-N-(2-Hydroxyhexadecanoyl)-aminooctadecan-1,3,4-triol ausgewählt wird.

11. Verwendung nach dem vorhergehenden Anspruch, dadurch gekennzeichnet, daß es sich bei der Verbindung der Formel (I) um 2-N-(2-Hydroxyhexadecanoyl)-aminooctadecan-1,3-diol oder 2-Oleoylaminooctadecan-1,3-diol handelt.

12. Verwendung nach einem der vorhergehenden Ansprüche zur Bekämpfung einer durch eine Krebsbehandlung, die aus einer Chemotherapie besteht, hervorgerufenen Alopezie.

13. Kosmetische oder pharmazeutische Zusammensetzung, die in einem kosmetisch oder pharmazeutisch akzeptablen Medium mindestens eine Verbindung, die wie in einem der Ansprüche 1 bis 11 definiert ist, und mindestens ein Mittel enthält, das unter antibakteriellen Mitteln, antiparasitären Mitteln, Antimykotika, antiviralen Mitteln, entzündungshemmenden Mitteln, Antipruriginosa, Anästhetika, Keratolytika, Mitteln gegen freie Radikale, Antiseborrhöika, Antischuppenmitteln, Aknemitteln, Mitteln, die die Differenzierung und/oder die Proliferation und/oder die Pigmentierung der Haut modulieren, den Antagonisten der Substanz P, von CGRP oder Bradykinin und den Inhibitoren der NO-Synthase ausgewählt ist.

14. Zusammensetzung nach dem vorhergehenden Anspruch, dadurch gekennzeichnet, daß der Antagonist der Substanz P, von CGRP oder Bradykinin oder der Inhibitor der NO-Synthase in einer Menge von 0,000001 bis 20 % des Gesamtgewichts der Zusammensetzung und insbesondere in einer Menge von 0,0001 bis 15 % des Gesamtgewichts der Zusammensetzung verwendet wird.

15. Verfahren zur kosmetischen Behandlung der Haare und/oder der Kopfhaut, das dadurch gekennzeichnet ist, daß eine kosmetische Zusammensetzung, die wie in einem der Ansprüche 13 und 14 definiert ist, auf die Haare und/oder die Kopfhaut aufgetragen wird, daR diese Zusammensetzung mit den Haaren und/oder der Kopfhaut in Kontakt gehalten wird und gegebenenfalls ausgespült wird.
